# EUROPEAN PATENT SPECIFICATION

(11) **EP 3 038 525 B1**
(45) Date of publication and mention of the grant of the patent: **10.05.2017**
(21) Application number: 14755802.7
(22) Date of filing: 19.08.2014
(51) Int. Cl.: A61B 5/05, G01R 33/38, H01F 7/06

(54) **COIL ARRANGEMENT OF A MPI SYSTEM OR APPARATUS**
SPULENANORDNUNG EINES MPI-SYSTEMS ODER EINER MPI-VORRICHTUNG
AGENCEMENT DE BOBINES D'UN SYSTÈME OU APPAREIL MPI

(30) Priority: 30.08.2013 EP 13182444
(43) Date of publication of application: 06.07.2016
(73) Proprietor: Koninklijke Philips N.V., 5656 AE Eindhoven (NL)
(72) Inventor: SCHMALE, Ingo, NL-5656 AE Eindhoven (NL)
(74) Representative: Claveau, Olivier
(86) International application number: PCT/EP2014/067607
(87) International publication number: WO 2015/028343

(56) References cited:
- WO-A1-2013/080145
- MARLITT ERBE ET AL: "Experimental Validation of an Assembly of Optimized Curved Rectangular Coils for the Use in Dynamic Field Free Line Magnetic Particle Imaging", CURRENT MEDICAL IMAGING REVIEWS, BENTHAM SCIENCE PUBLISHERS LTD., BUSSUM, NL, vol. 9, no. 2, 1 May 2013 (2013-05-01), pages 89-95, XP009176370, ISSN: 1573-4056

## Description

### FIELD OF THE INVENTION

The present invention relates to an apparatus for influencing and/or detecting magnetic particles in a field of view, in particular a magnetic particle imaging apparatus. Further, the present invention relates to a coil arrangement, in particular for use in such a magnetic particle imaging apparatus.

### BACKGROUND OF THE INVENTION

Magnetic Particle Imaging (MPI) is an emerging medical imaging modality. The first versions of MPI were two-dimensional in that they produced two-dimensional images. Newer versions are three-dimensional (3D). A four-dimensional image of a non-static object can be created by combining a temporal sequence of 3D images to a movie, provided the object does not significantly change during the data acquisition for a single 3D image.

MPI is a reconstructive imaging method, like Computed Tomography (CT) or Magnetic Resonance Imaging (MRI). Accordingly, an MP image of an object's volume of interest is generated in two steps. The first step, referred to as data acquisition, is performed using an MPI scanner. The MPI scanner has means to generate a static magnetic gradient field, called the "selection field", which has a (single or more) field-free point(s) (FFP(s)) or a field-free line (FFL) at the isocenter of the scanner. Moreover, this FFP (or the FFL; mentioning "FFP" in the following shall generally be understood as meaning FFP or FFL) is surrounded by a first sub-zone with a low magnetic field strength, which is in turn surrounded by a second sub-zone with a higher magnetic field strength. In addition, the scanner has means to generate a time-dependent, typically spatially nearly homogeneous magnetic field. Actually, this field is obtained by superposing a rapidly changing field with a small amplitude, called the "drive field", and optionally a slowly varying field with a large amplitude, called the "focus field". By adding the time-dependent drive field and optional focus field to the static selection field, the FFP may be moved along a predetermined FFP trajectory throughout a "volume of scanning" surrounding the isocenter. The scanner also has an arrangement of one or more, e.g. three, receive coils and can record any voltages induced in these coils. For the data acquisition, the object to be imaged is placed in the scanner such that the object's volume of interest is enclosed by the scanner's field of view, which is a subset of the volume of scanning.

The object contains magnetic nanoparticles or other magnetic non-linear materials; if the object is an animal or a patient, a tracer containing such particles may be administered to the animal or patient prior to the scan. During the data acquisition, the MPI scanner moves the FFP along a deliberately chosen trajectory that traces out / covers the volume of scanning, or at least the field of view. The magnetic nanoparticles within the object experience a changing magnetic field and respond by changing their magnetization. The changing magnetization of the nanoparticles induces a time-dependent voltage in each of the receive coils. This voltage is sampled in a receiver associated with the receive coil. The samples output by the receivers are recorded and constitute the acquired data. The parameters that control the details of the data acquisition make up the "scan protocol".

In the second step of the image generation, referred to as image reconstruction, the image is computed, or reconstructed, from the data acquired in the first step. The image is typically a discrete 3D array of data that represents a sampled approximation to the position-dependent concentration of the magnetic nanoparticles in the field of view. The reconstruction is generally performed by a computer, which executes a suitable computer program. Computer and computer program realize a reconstruction algorithm. The reconstruction algorithm is based on a mathematical model of the data acquisition. As with all reconstructive imaging methods, this model can be formulated as an integral operator that acts on the acquired data; the reconstruction algorithm tries to undo, to the extent possible, the action of the model.

Such an MPI apparatus and method have the advantage that they can be used to examine arbitrary examination objects - e.g. human bodies - in a non-destructive manner and with a high spatial resolution, both close to the surface and remote from the surface of the examination object. Such an apparatus and method are generally known and have been first described in DE 101 51 778 A1 and in Gleich, B. and Weizenecker, J. (2005), "Tomographic imaging using the nonlinear response of magnetic particles" in Nature, vol. 435, pp. 1214-1217, in which also the reconstruction principle is generally described. The apparatus and method for magnetic particle imaging (MPI) described in that publication take advantage of the non-linear magnetization curve of small magnetic particles.

Drive coils are needed in MPI to generate the rapidly changing magnetic field (f ∼ 25kHz ... 200kHz or even higher), which has a typical amplitude of 20mT peak or less. The energy stored in the bore is proportional to the volume, hence rises with the third dimension of the radius. For a human size application, with a bore diameter of approximately 40cm (for a first experimental demonstrator and more for future products), the energy is around 10J (peak). The reactive power is the product of this times the angular frequency ω = 2* pi * f, so P_{react} ∼ 2 MW. This reactive power can be oscillated between magnetic field in the coil and electric field in the series capacitors by any product of current and voltage. As a typical example, Uₚₖ ~ 15kV, Iₚₖ ∼250A, both of which are challenging to operate.

Therefore the power needed in such systems has typically a very high value, and an optimization of its use can thus significantly reduce the power consumption costs and increase the security of the patients.

### SUMMARY OF THE INVENTION

WO2013/080145 A1 discloses a coil arrangement according to the preamble of claim 1. It is an object of the present invention to provide an apparatus for influencing and/or detecting magnetic particles in a field of view, i.e. an MPI apparatus, that enables the examination of such larger subjects (human beings, animals), in particular for adult human beings. Further, it is an object of the present invention to provide a coil arrangement which is more suitable for the examination of larger subjects (human beings, animals), in particular for adult human beings, by use of an MPI apparatus.

The invention is defined by the independent claim.

Preferred embodiments of the invention are defined in the dependent claims. It shall be understood that the claimed apparatus and the claimed coil arrangement have similar and/or identical preferred embodiments as defined in the dependent claims.

For sake of simplicity, and without any limitation whatsoever, in the following section of this specification, "cable" will refer to said "major cable" and "wires" will refer to said "minor cables or wires".

The patient's chest/trunk is generally placed inside the drive field coil arrangement which typically comprises one or several drive field coils (generally one coil or coil pair per one of the three spatial directions). To this end, the patient might actually slide into the generator by means of a patient support. The drive field coils occupy space between the patient and the selection field elements, which generally comprises selection field coils and/or permanent magnets which are arranged above and below the patient forming an open structure in a similar way as known from an open MRI apparatus. There are various trade-offs for the space between the upper and lower half of the selection field elements.

According to the present invention the drive field coil arrangement comprising one or various drive field coils has a maximum internal bore size (extending around said central longitudinal axis) allowing the patient to comfortably slide in. Further, the outer diameter is, at least in the direction facing the selection field elements, as small as possible allowing other components of the apparatus, in particular the selection field elements and preferably provided focus field coils to be arranged as close as possible to the patient. This is achieved according to the present invention by providing that at least one drive field coil, preferably all drive field coils, are slim at positions adjacent the selection field elements compared to positions not facing the selection field elements. In other words, the ratio of height to width is made low to make the cable and thus the drive coil slim at a certain position and the ratio of height to width is made high to make the cable and thus the drive coil thicker at a certain position.

In an embodiment in which the selection field elements are arranged above and below the patient, the at least one drive field coil is thus made slim in the vertical direction at the positions above and below the patient, while it is less slim at the positions on the left and right side of the patient. For this purpose the cable forming the at least one drive field coil does not, as conventionally, have a fixed cross-sections having a fixed shape but at least the shape of the cross-section changes along the longitudinal direction of the cable, while preferably the cross-section (i.e. the area of the cross-section) is kept constant.

In this context it shall be noted that there are various embodiments of drive field coils, in particular solenoid coils, which complete surrounds the field of view in an angular range of 360°, and saddle coils, which only surround the field of view in a smaller angular range of less than 180°, e.g. in the range of 90° to 160°. The angular sub-ranges are to be understood as portions of the respective (total) angular range and can be as small as only a few degrees (i.e. only a certain position). Generally, a sub-range is to be understood as an angular range between 5° and 90°, preferably between 15° and 75°.

In an embodiment the first angular sub-range is shifted by an angle in the range of 75° to 105°, in particular by an angle of substantially 90°, with respect to the second angular sub-range. Thus, at the sides of the patient (in particular under the axles when the apparatus is used for heart imaging) the cable is made thicker but with smaller width compared to the area above the chest and below the back of the patient where the cable is made thinner but with larger width.

In another embodiment the plurality of wires are arranged such that the ratio of height to width of the cable's cross-section has a first substantially identical value in oppositely arranged first and third angular sub-ranges (e.g. above and below the patient), which is different from a second substantially identical value in oppositely arranged second and fourth angular sub-ranges (e.g. at the sides of the patient). Thus, in the desired directions space can be saved.

This is preferably further achieved in an embodiment according to which the first angular sub-range is arranged facing a selection field element and the value of the ratio of height to width of the cable's cross-section is smaller in the first angular sub-range than in the second angular sub-range.

Preferably, multiple windings of the cable are arranged adjacent to each other in a z-direction substantially perpendicular to the longitudinal direction of the cable, wherein said windings are arranged closer together in the second angular sub-range than in the first angular sub-range. This is particular important if space in the z-direction, which corresponds to the longitudinal axis of the patient, is short, e.g. under the axles of the patient.

In the first angular sub-range the positions of the windings are displaced with respect to the positions of the windings in the second angular sub-range according to another preferred embodiment. In this way it is possible to design the peak of the coils sensitivity to be nearer to or ideally at a particular region of interest, e.g. the heart of the patient.

As explained above, the drive field coils are used to create high frequency (25 kHz up to 100 kHz or higher) magnetic drive fields for activating magnetic particles in the body in view of their detection for imaging purpose. Conventionally, drive field coils are realised with many windings, leading to a high inductance. However, this conventional design cannot be used anymore for the human-size MPI apparatus, as the voltage (e.g. 40kVpk) is far too high and will accordingly hardly comply with the medical instrumentation standard (IEC 60601-1). In a preferred embodiment said plurality of wires are twisted one ot the other along the cable (in other words around the longitudinal axis of the cable), in particular as a Rutherford cable. This solution provides for an inductance with fewer windings made of a thicker, so-called "Rutherford"-like cable, which has a flat appearance, and in which each wire sees each position equally often. Such a Rutherford cable mimics a perfect RF-Litz wire. Further, said wires are preferably Litz wires comprising a plurality of strands to have a low-loss cable type.

As already mentioned the at least one drive field coil is a solenoid coil or a saddle coil. Preferably, said drive field coils forming a drive field coil arrangement, comprise two pairs of saddle coils arranged around a central symmetry axis perpendicular to said central longitudinal axis and a solenoid coil arranged around said central symmetry axis. Some or all of the drive field coils are designed as explained above for the at least one drive field coil.

In another embodiment the drive elements comprise a carrier structure carrying said drive field coils on its outer surface and/or its inner surface, preferably comprising grooves for receiving cables forming said drive field coils. Thus, the drive field coils have a fixed structure and are pre-formed. In an alternative embodiment the drive field coils are flexible and can be placed around the patient as needed.

Advantageously, said at least one drive field coil is a saddle coil, wherein the plurality of wires forming the cable of said at least one drive field coil are twisted one to the other along the cable (in other words around the longitudinal axis of the cable), in particular as a Rutherford cable, while the cable is arranged on the outer surface or inner surface of the carrier structure to form said at least one drive field coil. Thus, the cable of the at least one drive field coil is not pre-formed on a workbench and then brought into the right form, which might be difficult in case of a saddle coil since the cable can only be bent in one direction but may be hard to bend in the other direction. Thus, the cable is formed (i.e. the wires are twisted to form the cable) on the fly while the cable is brought into the right form for forming the at least one drive field coil which makes it easier to bend the cable in the right form.

In still another embodiment the apparatus further comprises a connection cable for connecting the at least one drive field coil with the drive field signal generator unit, said connection cable having an unvaried cross-section and a transition unit for connecting the cable forming said at least one drive field coil with the connection cable.

In another aspect of the present disclosure an apparatus for influencing and/or detecting magnetic particles in a field of view is presented, which apparatus comprises:
- selection elements comprising a selection field signal generator unit and selection field elements for generating a magnetic selection field having a pattern in space of its magnetic field strength such that a first sub-zone having a low magnetic field strength where the magnetization of the magnetic particles is not saturated and a second sub-zone having a higher magnetic field strength where the magnetization of the magnetic particles is saturated are formed in the field of view,
- drive elements comprising a drive field signal generator unit and at least one drive field coil for changing the position in space of the two sub-zones in the field of view by means of a magnetic drive field so that the magnetization of the magnetic material changes locally, said at least one drive field coil being arranged generally around a central longitudinal axis passing through the field of view,
wherein at least one drive field coil is formed by a major cable arranged around the central longitudinal axis, wherein the major cable comprises a plurality of Litz wires comprising a plurality of strands, said Litz wires being twisted one to the other along the major cable, in particular as Rutherford cable.

The apparatus according to this aspect primarily provides the advantages explained above in the context of Rutherford cables.

For receiving detection signals for determining the distribution of magnetic particles within the examination area and, thus, for generating images of the examination area, e.g. of the heart region of a patient, the apparatus further comprises a receiving means comprising at least one signal receiving unit and at least one receiving coil for acquiring detection signals, which detection signals depend on the magnetization in the field of view, which magnetization is influenced by the change in the position in space of the first and second sub-zone.

It is preferably proposed that the MPI apparatus employs combined selection-and-focus field coils, which is based on the idea to combine focus field coils and the selection field coils that are generally provided as separate coils in the known MPI apparatus into a combined set of selection-and-focus field coils. Hence, a single current is provided to each of said coils rather than separate currents as conventionally provided to each focus field coil and each selection field coil. The single currents can thus be regarded as two superposed currents for focus field generation and selection field generation. The desired location and movement of the field of view within the examination area can be easily changed by controlling the currents to the various coils. Not all selection-and-focus field coils must, however, always be provided with control currents, as some coils are only needed for certain movements of the field of view.

The proposed apparatus further provides more freedom of how and where to arrange the coils with respect to the examination area in which the subject is place. It is particularly possible with this arrangement to build an open scanner that is easily accessible both by the patient and by doctors or medical personnel, e.g. a surgeon during an intervention.

With such an apparatus the magnetic gradient field (i.e. the magnetic selection field) is generated with a spatial distribution of the magnetic field strength such that the field of view comprises a first sub-area with lower magnetic field strength (e.g. the FFP), the lower magnetic field strength being adapted such that the magnetization of the magnetic particles located in the first sub-area is not saturated, and a second sub-area with a higher magnetic field strength, the higher magnetic field strength being adapted such that the magnetization of the magnetic particles located in the second sub-area is saturated. Due to the non-linearity of the magnetization characteristic curve of the magnetic particles the magnetization and thereby the magnetic field generated by the magnetic particles shows higher harmonics, which, for example, can be detected by a detection coil. The evaluated signals (the higher harmonics of the signals) contain information about the spatial distribution of the magnetic particles, which again can be used e.g. for medical imaging, for the visualization of the spatial distribution of the magnetic particles and/or for other applications.

The MPI apparatus according to the present invention are based on a new physical principle (i.e. the principle referred to as MPI) that is different from other known conventional medical imaging techniques, as for example nuclear magnetic resonance (NMR). In particular, this new MPI-principle, does, in contrast to NMR, not exploit the influence of the material on the magnetic resonance characteristics of protons, but rather directly detects the magnetization of the magnetic material by exploiting the non-linearity of the magnetization characteristic curve. In particular, the MPI-technique exploits the higher harmonics of the generated magnetic signals which result from the non-linearity of the magnetization characteristic curve in the area where the magnetization changes from the non-saturated to the saturated state.

The drive field coils are preferably arranged in the area between said first inner selection-and-focus field coils of the two sets of selection-and-focus field coils. The drive field coils may be designed such that they are (fixedly or movable) arranged between the two sets of selection-and-focus field coils. In other embodiments, the drive field coils are somewhat flexible and can be arranged on the desired portion of the patient's body before the patient is placed inside the examination area.

### BRIEF DESCRIPTION OF THE DRAWINGS

These and other aspects of the invention will be apparent from and elucidated with reference to the embodiment(s) described hereinafter. In the following drawings
Fig. 1 shows a first embodiment of an MPI apparatus,
Fig. 2 shows an example of the selection field pattern produced by an apparatus as shown in Fig. 1,
Fig. 3 shows a second embodiment of an MPI apparatus,
Fig. 4 shows a third and a fourth embodiment of an MPI apparatus,
Fig. 5 shows a block diagram of an MPI apparatus according to the present invention,
Fig. 6 shows two views of a first embodiment of a drive field coil according to the present invention,
Fig. 7 shows two views of a second embodiment of a drive field coil according to the present invention,
Fig. 8 shows a perspective view and a cross-section through an embodiment of a cable for use in a drive field coil according to the present invention,
Fig. 9 shows how the cable shall be flat around the bore,
Fig. 10 shows an embodiment of a saddle coil pair for use as drive field coil according to another embodiment of the present invention, and
Fig. 11 shows a connection cable for externally connecting a drive field coil.

### DETAILED DESCRIPTION OF THE INVENTION

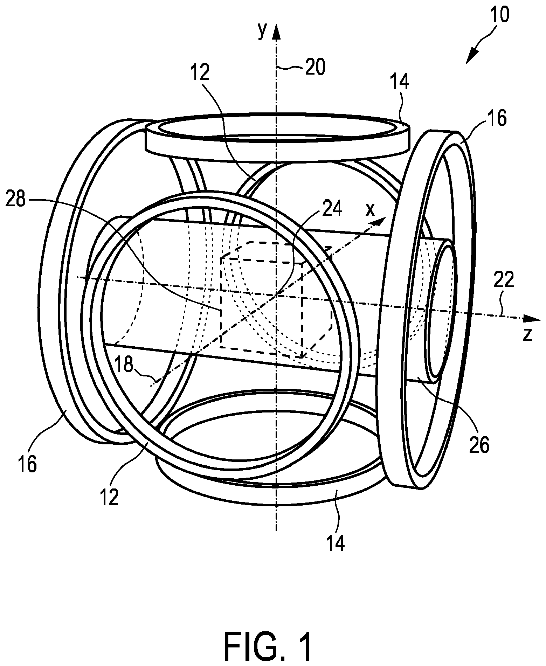

Before the details of the present invention shall be explained, basics of magnetic particle imaging shall be explained in detail with reference to Figs. 1 to 4. In particular, four embodiments of an MPI scanner for medical diagnostics will be described. An informal description of the data acquisition will also be given. The similarities and differences between the different embodiments will be pointed out. Generally, the present invention can be used in all these different embodiments of an MPI apparatus.

The first embodiment 10 of an MPI scanner shown in Fig. 1 has three pairs 12, 14, 16 of coaxial parallel circular coils, these coil pairs being arranged as illustrated in Fig. 1. These coil pairs 12, 14, 16 serve to generate the selection field as well as the drive and focus fields. The axes 18, 20, 22 of the three coil pairs 12, 14, 16 are mutually orthogonal and meet in a single point, designated the isocenter 24 of the MPI scanner 10. In addition, these axes 18, 20, 22 serve as the axes of a 3D Cartesian x-y-z coordinate system attached to the isocenter 24. The vertical axis 20 is nominated the y-axis, so that the x- and z-axes are horizontal. The coil pairs 12, 14, 16 are named after their axes. For example, the y-coil pair 14 is formed by the coils at the top and the bottom of the scanner. Moreover, the coil with the positive (negative) y-coordinate is called the y -coil (y⁻-coil), and similarly for the remaining coils. When more convenient, the coordinate axes and the coils shall be labelled with x₁, x₂, and x₃, rather than with x, y, and z.

The scanner 10 can be set to direct a predetermined, time-dependent electric current through each of these coils 12, 14, 16, and in either direction. If the current flows clockwise around a coil when seen along this coil's axis, it will be taken as positive, otherwise as negative. To generate the static selection field, a constant positive current I^{s} is made to flow through the z⁺-coil, and the current -Is is made to flow through the z⁻-coil. The z-coil pair 16 then acts as an anti-parallel circular coil pair.

It should be noted here that the arrangement of the axes and the nomenclature given to the axes in this embodiment is just an example and might also be different in other embodiments. For instance, in practical embodiments the vertical axis is often considered as the z-axis rather than the y-axis as in the present embodiment. This, however, does not generally change the function and operation of the device and the effect of the present invention.

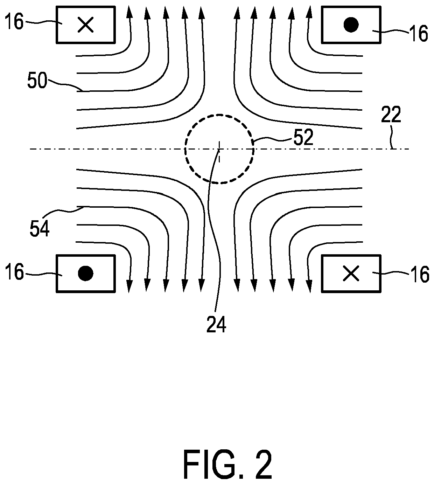

The magnetic selection field, which is generally a magnetic gradient field, is represented in Fig. 2 by the field lines 50. It has a substantially constant gradient in the direction of the (e.g. horizontal) z-axis 22 of the z-coil pair 16 generating the selection field and reaches the value zero in the isocenter 24 on this axis 22. Starting from this field-free point (not individually shown in Fig. 2), the field strength of the magnetic selection field 50 increases in all three spatial directions as the distance increases from the field-free point. In a first sub-zone or region 52 which is denoted by a dashed line around the isocenter 24 the field strength is so small that the magnetization of particles present in that first sub-zone 52 is not saturated, whereas the magnetization of particles present in a second sub-zone 54 (outside the region 52) is in a state of saturation. In the second sub-zone 54 (i.e. in the residual part of the scanner's field of view 28 outside of the first sub-zone 52) the magnetic field strength of the selection field is sufficiently strong to keep the magnetic particles in a state of saturation.

By changing the position of the two sub-zones 52, 54 (including the field-free point) within the field of view 28 the (overall) magnetization in the field of view 28 changes. By determining the magnetization in the field of view 28 or physical parameters influenced by the magnetization, information about the spatial distribution of the magnetic particles in the field of view 28 can be obtained. In order to change the relative spatial position of the two sub-zones 52, 54 (including the field-free point) in the field of view 28, further magnetic fields, i.e. the magnetic drive field, and, if applicable, the magnetic focus field, are superposed to the selection field 50.

To generate the drive field, a time dependent current I^{D}₁ is made to flow through both x-coils 12, a time dependent current I^{D}₂ through both y-coils 14, and a time dependent current I^{D}₃ through both z-coils 16. Thus, each of the three coil pairs acts as a parallel circular coil pair. Similarly, to generate the focus field, a time dependent current I^{F}₁ is made to flow through both x-coils 12, a current I^{F}₂ through both y-coils 14, and a current I^{F}₃ through both z-coils 16.

It should be noted that the z-coil pair 16 is special: It generates not only its share of the drive and focus fields, but also the selection field (of course, in other embodiments, separate coils may be provided). The current flowing through the z^{±}-coil is I^{D}₃ + I^{F}₃ ± I^{S}. The current flowing through the remaining two coil pairs 12, 14 is I^{D}ₖ + I^{F}ₖ, k = 1, 2. Because of their geometry and symmetry, the three coil pairs 12, 14, 16 are well decoupled. This is wanted.

Being generated by an anti-parallel circular coil pair, the selection field is rotationally symmetric about the z-axis, and its z-component is nearly linear in z and independent of x and y in a sizeable volume around the isocenter 24. In particular, the selection field has a single field-free point (FFP) at the isocenter. In contrast, the contributions to the drive and focus fields, which are generated by parallel circular coil pairs, are spatially nearly homogeneous in a sizeable volume around the isocenter 24 and parallel to the axis of the respective coil pair. The drive and focus fields jointly generated by all three parallel circular coil pairs are spatially nearly homogeneous and can be given any direction and strength, up to some maximum strength. The drive and focus fields are also time-dependent. The difference between the focus field and the drive field is that the focus field varies slowly in time and may have a large amplitude, while the drive field varies rapidly and has a small amplitude. There are physical and biomedical reasons to treat these fields differently. A rapidly varying field with a large amplitude would be difficult to generate and potentially hazardous to a patient.

In a practical embodiment the FFP can be considered as a mathematical point, at which the magnetic field is assumed to be zero. The magnetic field strength increases with increasing distance from the FFP, wherein the increase rate might be different for different directions (depending e.g. on the particular layout of the device). As long as the magnetic field strength is below the field strength required for bringing magnetic particles into the state of saturation, the particle actively contributes to the signal generation of the signal measured by the device; otherwise, the particles are saturated and do not generate any signal.

The embodiment 10 of the MPI scanner has at least one further pair, preferably three further pairs, of parallel circular coils, again oriented along the x-, y- and z-axes. These coil pairs, which are not shown in Fig. 1, serve as receive coils. As with the coil pairs 12, 14, 16 for the drive and focus fields, the magnetic field generated by a constant current flowing through one of these receive coil pairs is spatially nearly homogeneous within the field of view and parallel to the axis of the respective coil pair. The receive coils are supposed to be well decoupled. The time-dependent voltage induced in a receive coil is amplified and sampled by a receiver attached to this coil. More precisely, to cope with the enormous dynamic range of this signal, the receiver samples the difference between the received signal and a reference signal. The transfer function of the receiver is non-zero from zero Hertz ("DC") up to the frequency where the expected signal level drops below the noise level. Alternatively, the MPI scanner has no dedicated receive coils. Instead the drive field transmit coils are used as receive coils as is the case according to the present invention using combined drive-receiving coils.

The embodiment 10 of the MPI scanner shown in Fig. 1 has a cylindrical bore 26 along the z-axis 22, i.e. along the axis of the selection field. All coils are placed outside this bore 26. For the data acquisition, the patient (or object) to be imaged is placed in the bore 26 such that the patient's volume of interest - that volume of the patient (or object) that shall be imaged - is enclosed by the scanner's field of view 28 - that volume of the scanner whose contents the scanner can image. The patient (or object) is, for instance, placed on a patient table. The field of view 28 is a geometrically simple, isocentric volume in the interior of the bore 26, such as a cube, a ball, a cylinder or an arbitrary shape. A cubical field of view 28 is illustrated in Fig. 1.

The size of the first sub-zone 52 is dependent on the strength of the gradient of the magnetic selection field and on the field strength of the magnetic field required for saturation, which in turn depends on the magnetic particles. For a sufficient saturation of typical magnetic particles at a magnetic field strength of 80 A/m and a gradient (in a given space direction) of the field strength of the magnetic selection field amounting to 50x10³ A/m², the first sub-zone 52 in which the magnetization of the particles is not saturated has dimensions of about 1 mm (in the given space direction).

The patient's volume of interest is supposed to contain magnetic nanoparticles. Prior to the diagnostic imaging of, for example, a tumor, the magnetic particles are brought to the volume of interest, e.g. by means of a liquid comprising the magnetic particles which is injected into the body of the patient (object) or otherwise administered, e.g. orally, to the patient.

Generally, various ways for bringing the magnetic particles into the field of view exist. In particular, in case of a patient into whose body the magnetic particles are to be introduced, the magnetic particles can be administered by use of surgical and non-surgical methods, and there are both methods which require an expert (like a medical practitioner) and methods which do not require an expert, e.g. can be carried out by laypersons or persons of ordinary skill or the patient himself / herself. Among the surgical methods there are potentially non-risky and/or safe routine interventions, e.g. involving an invasive step like an injection of a tracer into a blood vessel (if such an injection is at all to be considered as a surgical method), i.e. interventions which do not require considerable professional medical expertise to be carried out and which do not involve serious health risks. Further, non-surgical methods like swallowing or inhalation can be applied.

Generally, the magnetic particles are pre-delivered or pre-administered before the actual steps of data acquisition are carried out. In embodiments, it is, however, also possible that further magnetic particles are delivered / administered into the field of view.

An embodiment of magnetic particles comprises, for example, a spherical substrate, for example, of glass which is provided with a soft-magnetic layer which has a thickness of, for example, 5 nm and consists, for example, of an iron-nickel alloy (for example, Permalloy). This layer may be covered, for example, by means of a coating layer which protects the particle against chemically and/or physically aggressive environments, e.g. acids. The magnetic field strength of the magnetic selection field 50 required for the saturation of the magnetization of such particles is dependent on various parameters, e.g. the diameter of the particles, the used magnetic material for the magnetic layer and other parameters.

In the case of e.g. a diameter of 10 µm with such magnetic particles, a magnetic field of approximately 800 A/m (corresponding approximately to a flux density of 1 mT) is then required, whereas in the case of a diameter of 100 µm a magnetic field of 80 A/m suffices. Even smaller values are obtained when a coating of a material having a lower saturation magnetization is chosen or when the thickness of the layer is reduced.

In practice, magnetic particles commercially available under the trade name Resovist (or similar magnetic particles) are often used, which have a core of magnetic material or are formed as a massive sphere and which have a diameter in the range of nanometers, e.g. 40 or 60 nm.

For further details of the generally usable magnetic particles and particle compositions, the corresponding parts of EP 1224542, WO 2004/091386, WO 2004/091390, WO 2004/091394, WO 2004/091395, WO 2004/091396, WO 2004/091397, WO 2004/091398, WO 2004/091408 are herewith referred to. In these documents more details of the MPI method in general can be found as well.

During the data acquisition, the x-, y-, and z-coil pairs 12, 14, 16 generate a position- and time-dependent magnetic field, the applied field. This is achieved by directing suitable currents through the field generating coils. In effect, the drive and focus fields push the selection field around such that the FFP moves along a preselected FFP trajectory that traces out the volume of scanning - a superset of the field of view. The applied field orientates the magnetic nanoparticles in the patient. As the applied field changes, the resulting magnetization changes too, though it responds nonlinearly to the applied field. The sum of the changing applied field and the changing magnetization induces a time-dependent voltage Vₖ across the terminals of the receive coil pair along the xₖ-axis. The associated receiver converts this voltage to a signal Sₖ, which it processes further.

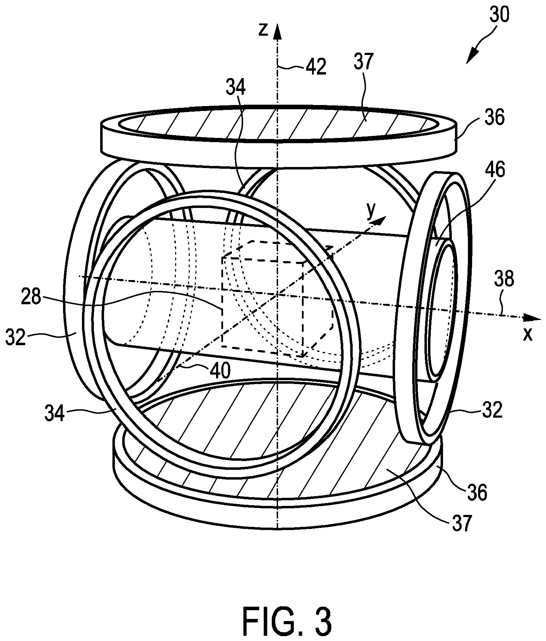

Like the first embodiment 10 shown in Fig. 1, the second embodiment 30 of the MPI scanner shown in Fig. 3 has three circular and mutually orthogonal coil pairs 32, 34, 36, but these coil pairs 32, 34, 36 generate the selection field and the focus field only. The z-coils 36, which again generate the selection field, are filled with ferromagnetic material 37. The z-axis 42 of this embodiment 30 is oriented vertically, while the x- and y-axes 38, 40 are oriented horizontally. The bore 46 of the scanner is parallel to the x-axis 38 and, thus, perpendicular to the axis 42 of the selection field. The drive field is generated by a solenoid (not shown) along the x-axis 38 and by pairs of saddle coils (not shown) along the two remaining axes 40, 42. These coils are wound around a tube which forms the bore. The drive field coils also serve as receive coils.

To give a few typical parameters of such an embodiment: The z-gradient of the selection field, G, has a strength of G/µ₀ = 2.5 T/m, where µ₀ is the vacuum permeability. The temporal frequency spectrum of the drive field is concentrated in a narrow band around 25 kHz (up to approximately 150 kHz). The useful frequency spectrum of the received signals lies between 50 kHz and 1 MHz (eventually up to approximately 15 MHz). The bore has a diameter of 120 mm. The biggest cube 28 that fits into the bore 46 has an edge length of 120 mm/√2 ≈ 84 mm.

Since the construction of field generating coils is generally known in the art, e.g. from the static B0 field of magnetic resonance imaging, this subject need not be further elaborated herein.

In an alternative embodiment for the generation of the selection field, permanent magnets (not shown) can be used. In the space between two poles of such (opposing) permanent magnets (not shown) there is formed a magnetic field which is similar to that shown in Fig. 2, that is, when the opposing poles have the same polarity. In another alternative embodiment, the selection field can be generated by a mixture of at least one permanent magnet and at least one coil.

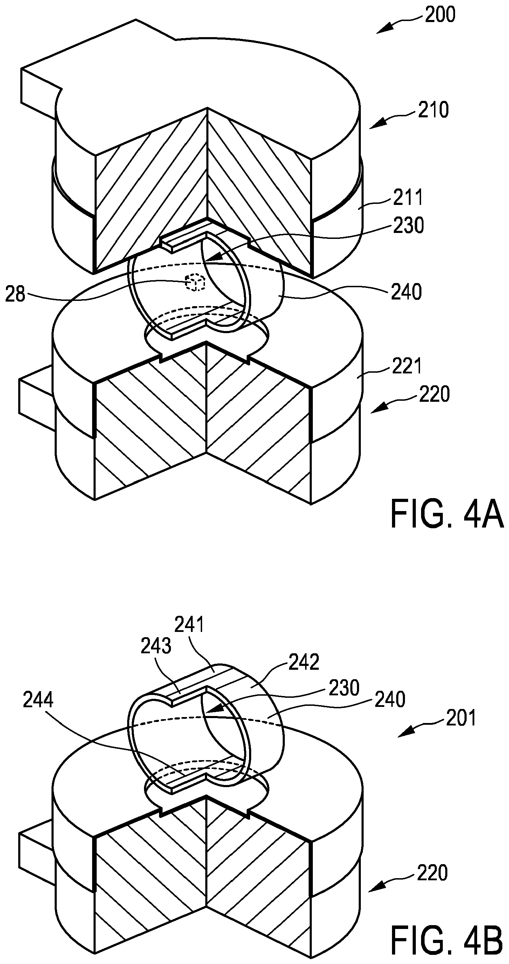

Fig. 4 shows two embodiments of the general outer layout of an MPI apparatus 200. Fig. 4A shows an embodiment of the proposed MPI apparatus 200 comprising two selection-and-focus field coil units 210, 220 which are basically identical and arranged on opposite sides of the examination area 230 formed between them. Further, a drive field coil unit 240 is arranged between the selection-and-focus field coil units 210, 220, which are placed around the area of interest of the patient (not shown). The selection-and-focus field coil units 210, 220 comprise several selection-and-focus field coils for generating a combined magnetic field representing the above-explained magnetic selection field and magnetic focus field. In particular, each selection-and-focus field coil unit 210, 220 comprises a, preferably identical, set of selection-and-focus field coils. Details of said selection-and-focus field coils will be explained below.

The drive field coil unit 240 comprises a number of drive field coils for generating a magnetic drive field. These drive field coils may comprise several pairs of drive field coils, in particular one pair of drive field coils for generating a magnetic field in each of the three directions in space. In an embodiment the drive field coil unit 240 comprises two pairs of saddle coils for two different directions in space and one solenoid coil for generating a magnetic field in the longitudinal axis of the patient.

The selection-and-focus field coil units 210, 220 are generally mounted to a holding unit (not shown) or the wall of room. Preferably, in case the selection-and-focus field coil units 210, 220 comprise pole shoes for carrying the respective coils, the holding unit does not only mechanically hold the selection-and-focus field coil unit 210, 220 but also provides a path for the magnetic flux that connects the pole shoes of the two selection-and-focus field coil units 210, 220.

As shown in Fig. 4a, the two selection-and-focus field coil units 210, 220 each include a shielding layer 211, 221 for shielding the selection-and-focus field coils from magnetic fields generated by the drive field coils of the drive field coil unit 240.

In the embodiment of the MPI apparatus 201 shown in Fig. 4B only a single selection-and-focus field coil unit 220 is provided as well as the drive field coil unit 240. Generally, a single selection-and-focus field coil unit is sufficient for generating the required combined magnetic selection and focus field. Said single selection-and-focus field coil unit 220 may thus be integrated into a (not shown) patient table on which a patient is placed for the examination. Preferably, the drive field coils of the drive field coil unit 240 may be arranged around the patient's body already in advance, e.g. as flexible coil elements. In another implementation, the drive field coil unit 240 can be opened, e.g. separable into two subunits 241, 242 as indicated by the separation lines 243, 244 shown in Fig. 4b in axial direction, so that the patient can be placed in between and the drive field coil subunits 241, 242 can then be coupled together.

In still further embodiments of the MPI apparatus, even more selection-and-focus field coil units may be provided which are preferably arranged according to a uniform distribution around the examination area 230. However, the more selection-and-focus field coil units are used, the more will the accessibility of the examination area for placing a patient therein and for accessing the patient itself during an examination by medical assistance or doctors be limited.

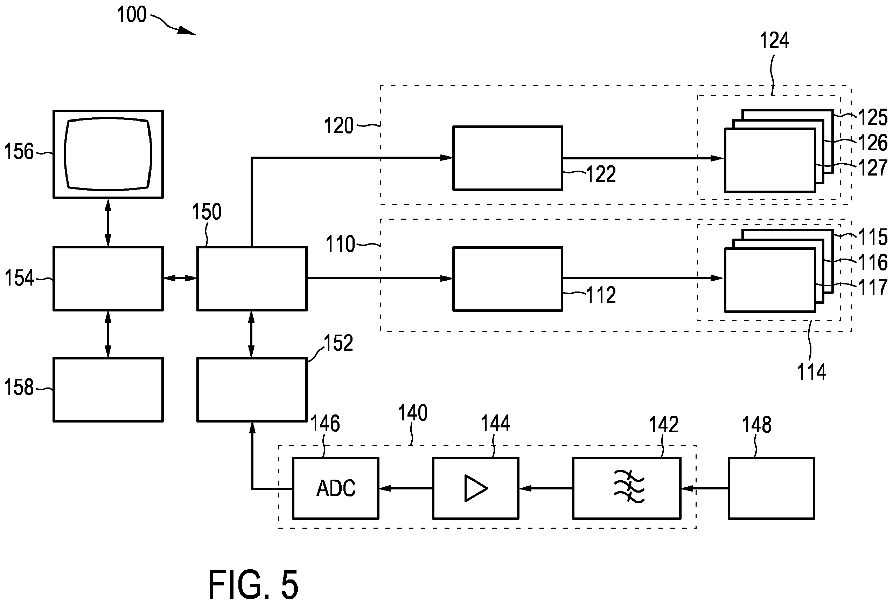

Fig. 5 shows a general block diagram of an MPI apparatus 100 according to the present invention. The general principles of magnetic particle imaging explained above are valid and applicable to this embodiment as well, unless otherwise specified.

The embodiment of the apparatus 100 shown in Fig. 5 comprises various coils for generating the desired magnetic fields. First, the coils and their functions in MPI shall be explained.

For generating the combined magnetic selection-and-focus field, selection-and-focus elements 110 are provided. The magnetic selection-and-focus field has a pattern in space of its magnetic field strength such that the first sub-zone (52 in Fig. 2) having a low magnetic field strength where the magnetization of the magnetic particles is not saturated and a second sub-zone (54 in Fig. 4) having a higher magnetic field strength where the magnetization of the magnetic particles is saturated are formed in the field of view 28, which is a small part of the examination area 230, which is conventionally achieved by use of the magnetic selection field. Further, by use the magnetic selection-and-focus field the position in space of the field of view 28 within the examination area 230 can be changed, as conventionally done by use of the magnetic focus field.

The selection-and-focus elements 110 comprises at least one set of selection-and-focus field coils 114 and a selection-and-focus field generator unit 112 for generating selection-and-focus field currents to be provided to said at least one set of selection-and-focus field coils 114 (representing one of the selection-and-focus field coil units 210, 220 shown in Figs. 4A, 4B) for controlling the generation of said magnetic selection-and-focus field. Preferably, a separate generator subunit is provided for each coil element (or each pair of coil elements) of the at least one set of selection-and-focus field coils 114. Said selection-and-focus field generator unit 112 comprises a controllable current source (generally including an amplifier) and a filter unit which provide the respective coil element with the field current to individually set the gradient strength and field strength of the contribution of each coil to the magnetic selection-and-focus field. It shall be noted that the filter unit can also be omitted.

For generating the magnetic drive field the apparatus 100 further comprises drive elements 120 comprising a drive field signal generator unit 122 and a set of drive field coils 124 (representing the drive coil unit 240 shown in Figs. 4A, 4B) for changing the position in space and/or size of the two sub-zones in the field of view by means of a magnetic drive field so that the magnetization of the magnetic material changes locally. As mentioned above said drive field coils 124 preferably comprise two pairs 125, 126 of oppositely arranged saddle coils and one solenoid coil 127. Other implementations, e.g. three pairs of coil elements, are also possible.

The drive field signal generator unit 122 preferably comprises a separate drive field signal generation subunit for each coil element (or at least each pair of coil elements) of said set of drive field coils 124. Said drive field signal generator unit 122 preferably comprises a drive field current source (preferably including a current amplifier) and a filter unit (which may also be omitted with the present invention) for providing a time-dependent drive field current to the respective drive field coil.

The selection-and-focus field signal generator unit 112 and the drive field signal generator unit 122 are preferably controlled by a control unit 150, which preferably controls the selection-and-focus field signal generator unit 112 such that the sum of the field strengths and the sum of the gradient strengths of all spatial points of the selection field is set at a predefined level. For this purpose the control unit 150 can also be provided with control instructions by a user according to the desired application of the MPI apparatus, which, however, is preferably omitted according to the present invention.

For using the MPI apparatus 100 for determining the spatial distribution of the magnetic particles in the examination area (or a region of interest in the examination area), particularly to obtain images of said region of interest, signal detection receiving means 148, in particular a receiving coil, and a signal receiving unit 140, which receives signals detected by said receiving means 148, are provided. Preferably, three receiving coils 148 and three receiving units 140 - one per receiving coil - are provided in practice, but more than three receiving coils and receiving units can be also used, in which case the acquired detection signals are not 3-dimensional but K-dimensional, with K being the number of receiving coils.

Said signal receiving unit 140 comprises a filter unit 142 for filtering the received detection signals. The aim of this filtering is to separate measured values, which are caused by the magnetization in the examination area which is influenced by the change in position of the two part-regions (52, 54), from other, interfering signals. To this end, the filter unit 142 may be designed for example such that signals which have temporal frequencies that are smaller than the temporal frequencies with which the receiving coil 148 is operated, or smaller than twice these temporal frequencies, do not pass the filter unit 142. The signals are then transmitted via an amplifier unit 144 to an analog/digital converter 146 (ADC).

The digitalized signals produced by the analog/digital converter 146 are fed to an image processing unit (also called reconstruction means) 152, which reconstructs the spatial distribution of the magnetic particles from these signals and the respective position which the first part-region 52 of the first magnetic field in the examination area assumed during receipt of the respective signal and which the image processing unit 152 obtains from the control unit 150. The reconstructed spatial distribution of the magnetic particles is finally transmitted via the control means 150 to a computer 154, which displays it on a monitor 156. Thus, an image can be displayed showing the distribution of magnetic particles in the field of view of the examination area.

In other applications of the MPI apparatus 100, e.g. for influencing the magnetic particles (for instance for a hyperthermia treatment) or for moving the magnetic particles (e.g. attached to a catheter for moving the catheter or attached to a medicament for moving the medicament to a certain location) the receiving means may also be omitted or simply not used.

Further, an input unit 158 may optionally be provided, for example a keyboard. A user may therefore be able to set the desired direction of the highest resolution and in turn receives the respective image of the region of action on the monitor 156. If the critical direction, in which the highest resolution is needed, deviates from the direction set first by the user, the user can still vary the direction manually in order to produce a further image with an improved imaging resolution. This resolution improvement process can also be operated automatically by the control unit 150 and the computer 154. The control unit 150 in this embodiment sets the gradient field in a first direction which is automatically estimated or set as start value by the user. The direction of the gradient field is then varied stepwise until the resolution of the thereby received images, which are compared by the computer 154, is maximal, respectively not improved anymore. The most critical direction can therefore be found respectively adapted automatically in order to receive the highest possible resolution.

In the known MPI apparatus the patient chest/trunk is placed inside the drive field coil unit. As explained above, the drive field coil unit typically comprises a solenoid coil made of several cables homogeneously wound around the cylinder-like bore in a straight, non-optimized way. For heart imaging this leads to non-optimal coil usage, hence more power is required to generate the requested drive field strength at the intended position of imaging (e.g. the heart).

WO 2013/080145 A1, particularly Fig. 19 discloses an MPI apparatus in which the solenoid coil comprises more cables having an increased cross-section area at the intended position of imaging (e.g. the heart). Nevertheless, connecting cables with different cross-section implies to have many lossy interface terminals leading to high loss for such a high-current, high-voltage and high-frequency MPI apparatus. Moreover, this locally larger cable cross-sections lead to a thicker drive field coil which takes away space from the selection coils or the selection- and focus-field coils, respectively, or from the patient, which should be avoided.

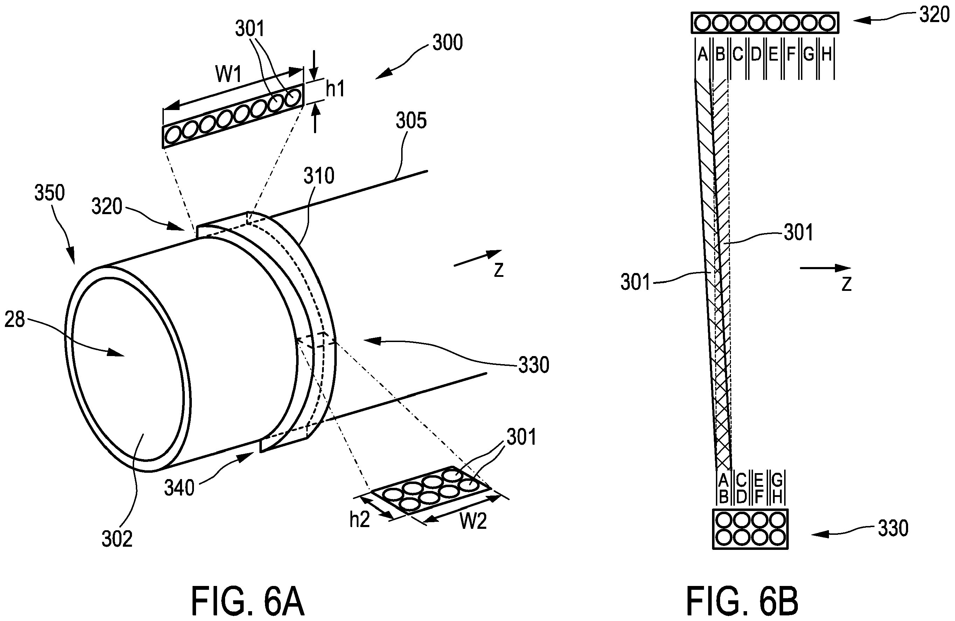

An embodiment of a drive field coil 300, in particular a solenoid coil, as used in an embodiment of an MPI apparatus according to the present invention is shown in Fig. 6A in a perspective view and, partially, in Fig. 6B in a cross-sectional view. According to this embodiment the drive field coil 300 is formed by a cable 310 (only one winding is shown for better visibility, but there are general several windings around the field of view 28), which is arranged at least in an angular range around the field of view 28 (here in the angular range of 360°). In this embodiment the cable 300 is arranged on the outer surface of a carrier structure 305, e.g. a tubular structure made e.g. of plastic material, which forms the bore 302 into which the patient is placed for examination. The cable 300 comprises a plurality of wires 301 forming said cable 300, which are arranged such that in a first angular sub-range 320 the ratio of height h1 to width w1 of the cable's cross-section is different than the ratio of height h2 to width w2 of the cable's cross-section in a second angular sub-range 330. In particular, h1<h2 and w1>w2 in this embodiment.

The sub-ranges 320, 330 are to be understood as angular ranges that are smaller than the complete angular range (here 360°) in which the cable 300 is arranged. For instance, the first sub-range 320, which is arranged here in the area of the top of the drive field coil 300, and the second sub-range 330, which is arranged here in the area of the side of the drive field coil 300, are in the range of only a few degrees (i.e. only a certain position), generally between 5° and 90°, preferably between 15° and 75°.

In other words, the cable 300 is wound around the cylinder-like patient bore 302 in a non-straight way, having its cross-section shape varying along its lengths. The relative positioning of the wires 301 of the cable 300 is varying one to the other depending on their angular locations around the cylindrical bore 302. This can particularly be seen in Fig. 6B showing how the (in this example eight) wires 301 in the first angular sub-range 320 are arranged next to each other in z-direction forming a thin but broad cable transform into a thicker but less broad cable in the second angular sub-range 330 where two layers of four wires 301 are stacked upon each other.

Preferably, not only at the top but also at the bottom (representing a third angular sub-range 340 arranged opposite to the first angular sub-range) the cable 300 has a broad but thin cross-section, and also on the other side opposite the second angular sub-range 330 in a fourth angular sub-range (350, not explicitly shown) the cable 300 has a less broad but thicker cross-section.

The variable cross-section shape thus allows reducing the thickness at the top and bottom location of the drive field coil 300 where the selection field coils (or the selection- and focus-field coils) are located. Preferably, on the top and bottom angular sub-ranges the thickness of the cable is minimized, while at the sides of the drive field coil (and the patient), where space is not that much of importance the cable is allowed to be thicker.

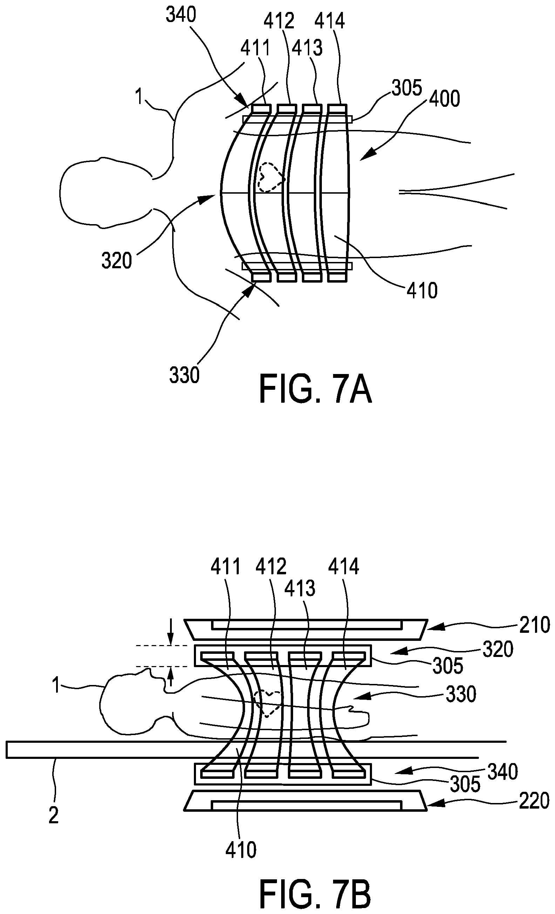

Another embodiment of a drive field coil 400, in particular a solenoid coil, as used in an embodiment of an MPI apparatus according to the present invention is shown in Fig. 7A in a top view and in Fig. 7B in a side view. In these figures four windings of the drive field coil 400 are shown, which are wound around the chest of the patient 1 who is lying on a patient support 2.

It should be noted that there are generally two possible positions for the arms, namely outside the drive field coil 400 (as shown in Fig. 7) or inside the drive field coil. The present invention is independent of the arm position.

The windings 411, 412, 413, 414 of the cable 410 forming the drive field coil 400 are arranged such that, in addition to the variation of the height and width along it length as explained above with reference to Fig. 6, the windings 411, 412, 413, 414 are arranged closer together in the second and fourth angular sub-ranges 330, 350 (i.e. under the axles) than in the first and third angular sub-range 320, 340 (i.e. above the chest and below the back). Thus, the total width of the drive field coil in the second and fourth angular sub-ranges 330, 350 is not only smaller because of the smaller width of the cable 410 there, but also because the windings are arranged close together.

The non-straight arrangement of the windings 411, 412, 413, 414 of the cable 410, intended for magnetic field generation in the z-direction allows designing the peak of the coil sensitivity to be nearer to or ideally at the heart of the patient 1. The windings are densely located beneath the axles (left/right of patient body), whilst they extend more towards the neck and chin (below and above the body).

The non-straight arrangement of the windings can be employed independently of the variable cross-section shape, but it is advantageous to combine both ideas as it allows to have smooth current density distribution along the drive field coil, which in turn translates into non-peaking induced currents in the patient and hence to a better tolerance with respect to peripheral nerve stimulation.

The same ideas can generally also be applied for the other drive field coils, which are preferably designed as saddle coil pairs. Also for such type of coils the cable can be designed to have a variable thickness to width ratio and/or a variable distance between the windings depending on the angular location.

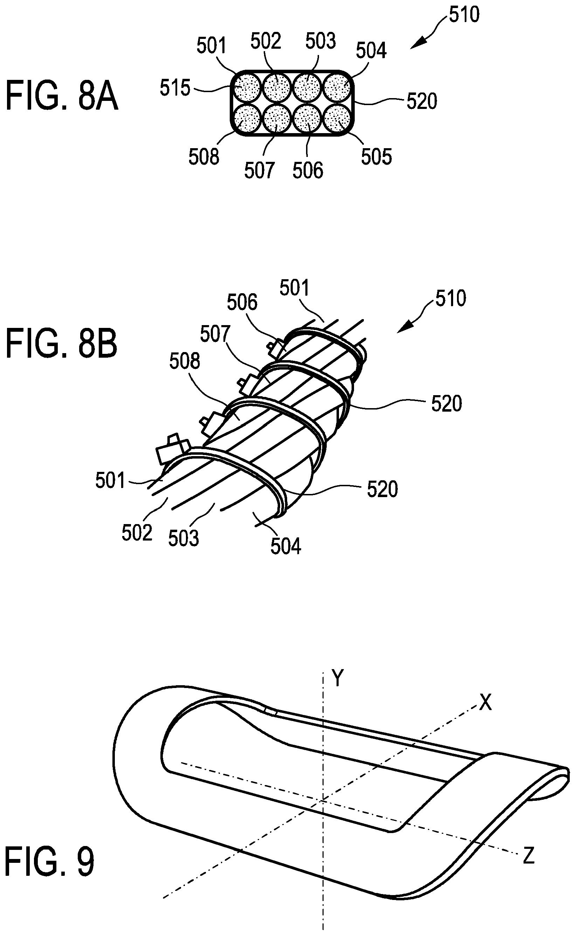

Fig. 8A shows a cross-section through an embodiment of a cable 510 for use in a drive field coil according to the present invention, for instance in a coil 300 or 400 shown above or in other embodiments of drive field coils. Fig. 8B shows a perspective view of this cable 510. The cable 510 comprises a plurality of Litz wires (in this example eight Litz wires 501-508) each comprising a plurality of strands 515 (e.g. 40000 strands with a diameter of 20µm). As shown in Fig. 8B said Litz wires 501-508 are twisted around the longitudinal axis of the cable 510, in particular as Rutherford cable.

The Litz wires 501-508 are, in this embodiment, held together by holding elements 520, e.g. cable binders such that the cable 510 has a flat appearance. Each Litz wire sees each position equally often so that the whole cable mimics a perfect large-cross section RF-Litz wire. From one holding element 520 to the next (e.g. approx. every 6cm) the Litz wires shift/rotate by one position.

Forming a Rutherford cable on the lab bench is generally not difficult, but shaping it into the form of (especially) saddle coil is difficult, especially forming the inner winding, with smallest bending radius. The challenge with flat Rutherford cables is, that it is elongated much more in one direction (left-right) than in the other (top-down). Therefore, bending is nearly impossible in the elongated direction, whilst easy in the other. It is mathematically provable that such a saddle coil structure can only be attached around a cylinder-like shape (i.e. the bore into which the patient is to be placed) if the cable "stands". This type is called a CPE (constant perimeter end coil). However, in order to have an overall flat drive field coil for an MPI apparatus with few windings, it must "lie". Fig. 9 shows a computer sketch of a flat Rutherford cable on top of cylindrical bore, forming the upper saddle coil, to show how the cable shall be flat around the bore.

In order to achieve this, it is proposed to use a different manufacturing process. The cable shall not be preassembled on the work bench, but in a special form, in which it is pre-bent while rotating it. Alternatively, it can be assembled directly around or on the bore. In both cases, grooves for placing the cable are preferably provided. Further, holding elements (fixtures like cable binders and clips) are preferably used.

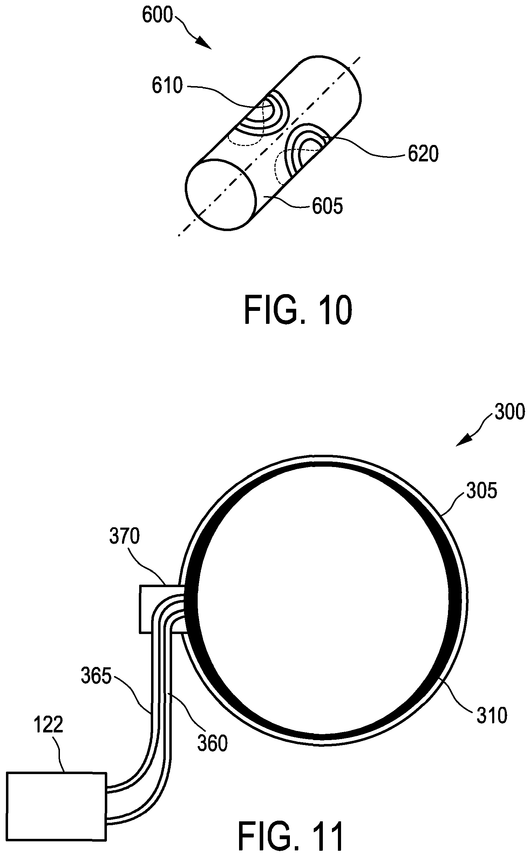

Thus, preferably for directions of the magnetic drive field orthogonal to the z-direction the drive field coils employ a Rutherford cable containing Litz wires with µm-thin strands, the cables being laid out on the bore according to a saddle coil pair configuration 600. Fig. 10 shows such a saddle coil pair configuration 600, a saddle coil 610, 620 comprising three windings of the cable 510, said three windings being coupled electrically preferably in parallel and formed on the inner or outer surface of a carrier 605.

The matching and tuning circuit can be realized such that the voltages at terminals are symmetric with respect to ground. E.g., if 10kVpk is the maximum across the inductor, then the terminals would be at +5kVpk and -5kVpk. There would be a virtual middle point at 0V.This feature is very useful, as it helps to reduce the voltages between the coils, as there are altogether three of them for the three spatial directions, at different frequencies. Without this symmetric realization, the maximum inter-coil voltage would be 2*10kVpk = 20kVpk, with this feature it is only 2*5kVpk=10kVpk. This helps to reduce insulating distances and materials within the drive field coils, and hence minimize space (which is then available for the patient).

Preferably, as shown in Fig. 11, two connection cables 360, 365 for connecting the at least one drive field coil with the drive field signal generator unit 122 and a transition unit 370 for connecting the cable 310 forming said at least one drive field coil 300 with the connection cable 360. One connection cable 360 is provided for the current to enter the drive field coil 300 and the other connection cable 365 is provided for the current to exit the drive field coil 300. The connection cables 360, 365 have a dual function: They carry electric current, but also surround the copper cables with a cooling liquid (preferably oil) to keep the connection cables 360, 365 cool.

Said connection cables 360, 365 are preferably Rutherford cables and have an unvaried (i.e. constant) cross-section. Thus, the transition unit 370 converts the connection cables 360, 365 into the cable 310 having the variable cross-section which may be achieved by connecting the various Litz wires of the cables via a connection board (not shown) to which the Litz wires are separately fixed. It is alternatively possible to use uninterrupted continuous Litz wire to form both the cable 310 within the drive field coil 300 and the two connection cables 360, 365, so that no connection board is needed.

The cable 310 is preferably wound to the inner surface of the carrier 305, wherein the winding process is preferably started from the middle of the cable (not the end of the cable), which may make it easier to bring the cable into the right form, in particular in case of forming a saddle coil.

Preferably, the saddle coils shall be coupled in parallel and not in series in order to keep the voltage low and to allow each coil to have a virtual middle point at 0V.

The various above explained aspects can each be used independently for single or all drive field coils, but are preferably used together in a preferred embodiment of an MPI apparatus according to the present invention. Preferably, all cables of all drive field coils are designed as Rutherford cables.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A coil arrangement for use in a magnetic particle imaging apparatus (100) comprising a major cable (310, 410, 510) arranged around a central longitudinal axis (z-axis), wherein the major cable (310, 410, 510) comprises a plurality of minor cables or wires (301, 501-508) forming said major cable **characterised in that** said plurality of minor cables or wires are positioned angularly differently around the central longitudinal axis (z-axis) such that in a first angular sub-range (320) the ratio of height to width of the major cable's cross-section is different than in a second angular sub-range (330).

2. The coil arrangement as claimed in claim 1,
wherein the first angular sub-range (320, 340) is offset by an angle in the range of 75° to 105°, in particular by an angle of substantially 90°, with respect to the second angular sub-range (330, 350).

3. The coil arrangement as claimed in claim 1,
wherein said minor cables or wires are further positioned angularly differently around the central longitudinal axis (z-axis) in third angular sub-range and in a fourth angular sub-range respectively opposed to the first and second angular sub-ranges around the central longitudinal axis (z-axis) such that the ratio of height to width of the major cable's cross-section has a first substantially similar value in the first and in the third angular sub-ranges (320, 340), and a second substantially similar value in the second and in the fourth angular sub-ranges (330, 350).

4. The coil arrangement as claimed in claim 1,
wherein the first angular sub-range (320, 340) is arranged facing a selection field element (220) and wherein the value of the ratio of height to width of the major cable's cross-section is smaller in the first angular sub-range (320, 340) than in the second angular sub-range (330, 350).

5. The coil arrangement as claimed in claim 4,
wherein multiple windings (301, 501-508) of the major cable are arranged adjacent to each other in a z-direction substantially orthogonal to the central longitudinal axis (z-axis), wherein said windings (301, 501-508) are arranged closer together in the second angular sub-range (330, 350) than in the first angular sub-range (320, 340).

6. The coil arrangement as claimed in claim 4 or 5, wherein, within the first angular sub-range (320, 340), the positions of the windings are angularly offset with respect to the positions of the windings within the second angular sub-range (330, 350).

7. The coil arrangement as claimed in claim 1, wherein said plurality of minor cables or wires (301, 501-508) are twisted one to the other along the major cable, in particular as Rutherford cable.

8. The coil arrangement as claimed in claim 1, wherein said plurality of minor cables or wires (301, 501-508) are Litz wires comprising a plurality of strands (515).

9. The coil arrangement as claimed in claim 1, wherein the major cable (310, 410, 510) comprises a plurality of Litz wires (301, 501-508) comprising a plurality of strands (515), said Litz wires being twisted one to the other along the major cable, in particular as Rutherford cable.

10. An apparatus (100) for influencing and/or detecting magnetic particles in a field of view (28), which apparatus comprises:
- selection elements comprising a selection field signal generator unit (110) and selection field elements (116) for generating a magnetic selection field (50) having a pattern in space of its magnetic field strength such that a first sub-zone (52) having a low magnetic field strength where the magnetization of the magnetic particles is not saturated and a second sub-zone (54) having a higher magnetic field strength where the magnetization of the magnetic particles is saturated are formed in the field of view (28),
- drive elements (120) comprising a drive field signal generator unit (122) and at least one drive field coil (124; 125, 126, 127) for changing the position in space of the two sub-zones (52, 54) in the field of view (28) by means of a magnetic drive field so that the magnetization of the magnetic material changes locally, said at least one drive field coil being arranged generally around a central longitudinal axis (z-axis), passing through the field of view (28),
wherein at least one drive field coil (300, 400, 600) is formed by the coil arrangement of any of claims 1 to 10.

11. The apparatus as claimed in claim 10, wherein the at least one drive field coil (300, 400, 600) is a solenoid coil or a saddle coil.

12. The apparatus as claimed in claim 10, wherein the drive elements comprise a carrier structure (305, 605) carrying the at least one drive field coil on its outer surface and/or its inner surface, preferably comprising at least one groove for receiving cables forming said drive field coil.

13. The apparatus as claimed in claim 12, wherein said at least one drive field coil (300, 400, 600) is a saddle coil, wherein the plurality of minor cables or wires (301, 501-508) forming the major cable of said at least one drive field coil are twisted one to the other along the major cable, in particular as Rutherford cable, while the major cable is arranged on the outer surface or inner surface of the carrier structure (305, 605) to form said at least one drive field coil.

14. The apparatus as claimed in claim 10, further comprising connection cables (360) for connecting the at least one drive field coil (300) with the drive field signal generator unit (122), said connection cable (360) having an unvaried general cross-section, and a transition unit (370) for connecting the cable forming said at least one drive field coil with the connection cable (360).

## Patentansprüche

1. Spulenanordnung zur Verwendung in einer MPI-Vorrichtung (100), ein Hauptkabel (310, 410, 510) umfassend, das um eine zentrale Längsachse (Z-Achse) herum angeordnet ist, wobei das Hauptkabel (310, 410, 510) eine Vielzahl von untergeordneten Kabeln oder Drähten (301, 501-508) umfasst, welche das besagte Hauptkabel bilden, **dadurch gekennzeichnet, dass** die besagte Vielzahl von untergeordneten Kabeln oder Drähten winkelig unterschiedlich um die zentrale Längsachse (Z-Achse) herum positioniert sind, sodass sich in einem ersten Winkel-Unterbereich (320) das Verhältnis von Höhe zu Breite des Querschnitts des Hauptkabels von jenem in einem zweiten Winkel-Unterbereich (330) unterscheidet.

2. Spulenanordnung nach Anspruch 1,
wobei der erste Winkel-Unterbereich (320, 340) durch einen Winkel im Bereich von 75° bis 105°, im Speziellen durch einen Winkel von im Wesentlichen 90° im Verhältnis zum zweiten Winkel-Unterbereich (330, 350) versetzt ist.

3. Spulenanordnung nach Anspruch 1,
wobei die besagten untergeordneten Kabel oder Drähte darüber hinaus winkelig unterschiedlich um die zentrale Längsachse (Z-Achse) herum in einem dritten Winkel-Unterbereich und in einem vierten Winkel-Unterbereich jeweils gegenüber den ersten und zweiten Winkel-Unterbereichen um die zentrale Längsachse (Z-Achse) herum positioniert sind, sodass das Verhältnis von Höhe zu Breite des Querschnitts des Hauptkabels einen ersten im Wesentlichen gleichen Wert im ersten und im dritten Winkel-Unterbereich (320, 340), und einen zweiten im Wesentlichen gleichen Wert im zweiten und im vierten Winkel-Unterbereichen (330, 350) aufweist.

4. Spulenanordnung nach Anspruch 1,
wobei der erste Winkel-Unterbereich (320, 340) einem Auswahlfeldelement (220) zugewandt angeordnet ist, und wobei der Wert des Verhältnisses von Höhe zu Breite des Querschnitts des Hauptkabels im ersten Winkel-Unterbereich (320, 340) kleiner ist, als im zweiten Winkel-Unterbereich (330, 350).

5. Spulenanordnung nach Anspruch 4,
wobei mehrfache Wicklungen (301, 501-508) des Hauptkabels angrenzend zueinander in eine Z-Richtung angeordnet sind, die im Wesentlichen orthogonal zur zentralen Längsachse (Z-Achse) verläuft, wobei die besagten Wicklungen (301, 501-508) im zweiten Winkel-Unterbereich (330, 350) näher aneinander angeordnet sind, als im ersten Winkel-Unterbereich (320, 340).

6. Spulenanordnung nach Anspruch 4 oder 5,
wobei innerhalb des ersten Winkel-Unterbereichs (320, 340) die Positionen der Wicklungen winkelig im Verhältnis zu den Positionen innerhalb des zweiten Winkel-Unterbereichs (330, 350) versetzt sind.

7. Spulenanordnung nach Anspruch 1,
wobei die besagte Vielzahl von untergeordneten Kabeln oder Drähten (301, 501-508) entlang des Hauptkabels, im Speziellen als Rutherford-Kabel, miteinander verdrillt sind.

8. Spulenanordnung nach Anspruch 1,
wobei die besagte Vielzahl von untergeordneten Kabeln oder Drähten (301, 501-508) Litzen-Drähte sind, die eine Vielzahl von Litzen (515) umfassen.

9. Spulenanordnung nach Anspruch 1,
wobei das Hauptkabel (310, 410, 510) eine Vielzahl von LitzenDrähten (301, 501-508) umfasst, die eine Vielzahl von Litzen (515) umfassen, wobei die besagten Litzen-Drähte entlang des Hauptkabels, im Speziellen als Rutherford-Kabel, miteinander verdrillt sind.

10. Vorrichtung (100) zum Beeinflussen und/ oder Erfassen von magnetischen Partikeln in einem Sichtfeld (28), wobei die Vorrichtung Folgendes umfasst:
- Auswahlelemente, eine Auswahlfeld-Signalerzeugungseinheit (110) und Auswahlfeldelemente (116) zum Erzeugen eines magnetischen Auswahlfeldes (50) umfassend, die ein Muster im Raum ihrer Magnetfeldstärke aufweisen, sodass eine erste Unterzone (52) mit einer niedrigen Magnetfeldstärke, wo die Magnetisierung der magnetischen Partikel nicht gesättigt ist, und eine zweite Unterzone (54) mit einer höheren Magnetfeldstärke, wo die Magnetisierung der magnetischen Partikel gesättigt ist, im Sichtfeld (28) gebildet werden,
- Antriebselemente (120), eine Antriebsfeld-Signalerzeugungseinheit (122) und zumindest eine Antriebsfeldspule (124; 125, 126, 127) zum Ändern der Position im Raum der beiden Unterzonen (52, 54) im Sichtfeld (28) durch ein magnetisches Antriebsfeld umfassend, sodass sich die Magnetisierung des magnetischen Materials lokal ändert, wobei die besagte zumindest eine Antriebsfeldspule im Allgemeinen um eine zentrale Längsachse (Z-Achse) herum angeordnet ist, welche durch das Sichtfeld (28) verläuft,
wobei zumindest eine Antriebsfeldspule (300, 400, 600) durch die Spulenanordnung nach irgendeinem der Ansprüche 1 bis 10 gebildet wird.

11. Vorrichtung nach Anspruch 10,
wobei die zumindest eine Antriebsfeldspule (300, 400, 600) eine Magnetspule oder eine Sattelspule ist.

12. Vorrichtung nach Anspruch 10,
wobei die Antriebselemente eine Trägerstruktur (305, 605) umfassen, welche die zumindest eine Antriebsfeldspule an ihrer Außenfläche und/ oder ihrer Innenfläche tragen, vorzugsweise zumindest eine Nut zur Aufnahme von Kabeln umfassend, welche die besagte Antriebsfeldspule bilden.

13. Vorrichtung nach Anspruch 12,
wobei die besagte zumindest eine Antriebsfeldspule (300, 400, 600) eine Sattelspule ist, wobei die Vielzahl von untergeordneten Kabeln oder Drähten (301, 501-508), welche das Hauptkabel der besagten zumindest einen Antriebsfeldspule bilden, entlang des Hauptkabels, im Speziellen als Rutherford-Kabel, miteinander verdrillt sind, während das Hauptkabel an der Außenfläche oder der Innenfläche der Trägerstruktur (305, 605) angeordnet ist, um die besagte zumindest eine Antriebsfeldspule zu bilden.

14. Vorrichtung nach Anspruch 10,
darüber hinaus Verbindungskabel (360) zum Verbinden der zumindest einen Antriebsfeldspule (300) mit der Antriebsfeld-Signalerzeugungseinheit (122) umfassend, wobei das besagte Verbindungskabel (360) einen unveränderten allgemeinen Querschnitt aufweist, und eine Übergangseinheit (370) zur Verbindung des Kabels, welches die besagte zumindest eine Antriebsfeldspule bildet, mit dem Verbindungskabel (360).

## Revendications

1. Agencement de bobine destiné à être utilisé dans un appareil d'imagerie à particules magnétiques (100) comprenant un grand câble (310, 410, 510) agencé autour d'un axe longitudinal central (axe z), dans lequel le grand câble (310, 410, 510) comprend une pluralité de petits câbles ou fils (301, 501 à 508) formant ledit grand câble, **caractérisé en ce que** ladite pluralité de petits câbles ou fils sont positionnés angulairement de façon différente autour de l'axe longitudinal central (axe z) de sorte que dans une première sous-plage angulaire (320), le rapport hauteur sur largeur de la section transversale du grand câble soit différent de celui dans une deuxième sous-plage angulaire (330).

2. Agencement de bobine selon la revendication 1,
dans lequel la première sous-plage angulaire (320, 340) est décalée d'un angle dans la plage de 75° à 105°, en particulier d'un angle de sensiblement 90°, par rapport à la deuxième sous-plage angulaire (330, 350).

3. Agencement de bobine selon la revendication 1,
dans lequel lesdits petits câbles ou fils sont en outre positionnés angulairement de façon différente autour de l'axe longitudinal central (axe z) dans une troisième sous-plage angulaire et dans une quatrième sous-plage angulaire respectivement opposées aux première et deuxième sous-plages angulaires autour de l'axe longitudinal central (axe z) de sorte que le rapport de hauteur sur largeur de la section transversale du grand câble ait une première valeur sensiblement similaire dans les première et troisième sous-plages angulaires (320, 340), et une seconde valeur sensiblement similaire dans les deuxième et quatrième sous-plages angulaires (330, 350).

4. Agencement de bobine selon la revendication 1,
dans lequel la première sous-plage angulaire (320, 340) est agencée face à un élément de champ de sélection (220) et dans lequel la valeur du rapport hauteur sur largeur de la section transversale du grand câble est plus petite dans la première sous-plage angulaire (320, 340) que dans la deuxième sous-plage angulaire (330, 350).

5. Agencement de bobine selon la revendication 4,
dans lequel de multiples enroulements (301, 501 à 508) du grand câble sont agencés adjacents les uns aux autres dans une direction z sensiblement orthogonale à l'axe longitudinal central (axe z), dans lequel lesdits enroulements (301, 501 à 508) sont agencés plus près les uns des autres dans la deuxième sous-plage angulaire (330, 350) que dans la première sous-plage angulaire (320, 340).

6. Agencement de bobine selon la revendication 4 ou 5,
dans lequel, dans la première sous-plage angulaire (320, 340), les positions des enroulements sont décalées angulairement par rapport aux positions des enroulements dans la deuxième sous-plage angulaire (330, 350).

7. Agencement de bobine selon la revendication 1,
dans lequel ladite pluralité de petits câbles ou fils (301, 501 à 508) sont torsadés les uns autour des autres le long du grand câble, en particulier sous forme de câble Rutherford.

8. Agencement de bobine selon la revendication 1,
dans lequel ladite pluralité de petits câbles ou fils (301, 501 à 508) sont des fils de Litz comprenant une pluralité de torons (515).

9. Agencement de bobine selon la revendication 1,
dans lequel le grand câble (310, 410, 510) comprend une pluralité de fils de Litz (301, 501 à 508) comprenant une pluralité de torons (515), lesdits fils de Litz étant torsadés les uns autour des autres le long du grand câble, en particulier sous forme de câble Rutherford.

10. Appareil (100) pour influencer et/ou détecter des particules magnétiques dans un champ de vision (28), lequel appareil comprend :
- des éléments de sélection comprenant une unité de génération de signal de champ de sélection (110) et des éléments de champ de sélection (116) pour générer un champ de sélection magnétique (50) ayant un motif dans l'espace de son intensité de champ magnétique de sorte qu'une première sous-zone (52) ayant une faible intensité de champ magnétique où l'aimantation des particules magnétiques n'est pas saturée et une seconde sous-zone (54) ayant une intensité de champ magnétique plus élevée où l'aimantation des particules magnétiques est saturée sont formées dans le champ de vision (28),
- des éléments d'attaque (120) comprenant une unité de génération de signal de champ d'attaque (122) et au moins une bobine de champ d'attaque (124 ; 125, 126, 127) pour changer la position dans l'espace des deux sous-zones (52, 54) dans le champ de vision (28) au moyen d'un champ d'attaque magnétique de sorte que l'aimantation du matériau magnétique change localement, ladite au moins une bobine de champ d'attaque étant agencée en général autour d'un axe longitudinal central (axe z), traversant le champ de vision (28),
dans lequel au moins une bobine de champ d'attaque (300, 400, 600) est formée par l'agencement de bobine de l'une quelconque des revendications 1 à 10.

11. Appareil selon la revendication 10,
dans lequel l'au moins une bobine de champ d'attaque (300, 400, 600) est une bobine de solénoïde ou une bobine à col.

12. Appareil selon la revendication 10,
dans lequel les éléments d'attaque comprennent une structure porteuse (305, 605) portant l'au moins une bobine de champ d'attaque sur sa surface extérieure et/ou sa surface intérieure, comprenant de préférence au moins une gorge pour recevoir des câbles formant ladite bobine de champ d'attaque.

13. Appareil selon la revendication 12,
dans lequel ladite au moins une bobine de champ d'attaque (300, 400, 600) est une bobine à col, dans lequel la pluralité de petits câbles ou fils (301, 501 à 508) formant le grand câble de ladite au moins une bobine de champ d'attaque sont torsadés les uns autour des autres le long du grand câble, en particulier sous forme de câble Rutherford, tandis que le grand câble est agencé sur la surface extérieure ou la surface intérieure de la structure porteuse (305, 605) pour former ladite au moins une bobine de champ d'attaque.

14. Appareil selon la revendication 10,
comprenant en outre des câbles de raccordement (360) pour raccorder l'au moins une bobine de champ d'attaque (300) à l'unité de génération de signal de champ d'attaque (122), ledit câble de raccordement (360) ayant une section transversale générale invariable, et une unité de transition (370) pour raccorder le câble formant ladite au moins une bobine de champ d'attaque au câble de raccordement (360).
